# EUROPÄISCHE PATENTSCHRIFT

(11) **EP 0 848 623 B1**
(45) Veröffentlichungstag und Bekanntmachung des Hinweises auf die Patenterteilung: **08.10.2003**
(21) Anmeldenummer: 96920674.7
(22) Anmeldetag: 05.07.1996
(51) Int. Cl.: A61M 5/315, A61M 5/24

(54) **INJEKTIONSGERÄT ZUM INJIZIEREN VON FLÜSSIGKEIT**
INJECTION DEVICE FOR INJECTION OF LIQUID
DISPOSITIF D'INJECTION DE LIQUIDE

(43) Veröffentlichungstag der Anmeldung: 24.06.1998
(73) Patentinhaber: Disetronic Licensing AG, 3400 Burgdorf (CH)
(72) Erfinder: MICHEL, Peter, CH-3400 Burgdorf (CH); NYDEGGER, Peter, CH-3068 Utzigen (CH); WEBER, Philipp, CH-3400 Burgdorf (CH)
(74) Vertreter: Schwabe - Sandmair - Marx
(86) Internationale Anmeldenummer: CH9600248
(87) Internationale Veröffentlichungsnummer: WO98001171

(56) Entgegenhaltungen:
- EP-A- 0 554 995
- WO-A-93/16740
- WO-A-96/07443

## Beschreibung

Die vorliegende Erfindung bezieht sich auf ein Injektionsgerät zum Injizieren von Flüssigkeit gemäss dem Oberbegriff des Anspruchs 1.

Spritzenförmige Injektionsgeräte zum Injizieren von Flüssigkeiten sind seit längerem bekannt. Sie besitzen einen hülsenförmigen Hauptkörper, der etwa in der Mitte zusammenschraubbar ist und sich in zwei Hauptbereiche einteilen lässt:
einen proximalen (dem Patienten abgewandten) die Abgabemechanik beinhaltenden Bereich, mindestens bestehend aus einem stabförmigen Abtriebsglied mit strukturierter Oberfläche, z.B. eine Schraubenspindel, einem zum Abtriebsglied passenden hohlzylindrischen Gegenelement mit einem strukturierten inneren Mantel, z.B. eine Schraubenmutter, und einem Bedienknopf; und einen distalen (dem Patienten zugewandten) Bereich, in welchem sich die zu verabreichende Flüssigkeit und ein beweglicher Kolben befinden.

Am distalen Ende des Hauptkörpers wird eine Nadel mit Nadelhalter aufgesetzt, welche den Ausfluss der Flüssigkeit aus dem Gerät ermöglicht; bekannte solche Nadeln sind z.B. PENFINE® Nadeln, wie in der WO 95/01812 beschrieben. Verbindungsglied zwischen dem proximalen und distalen Bereich des Hauptkörpers ist das Abtriebsglied, welches den Kolben um die gewählte Dosis in die distale Richtung schiebt, was zum Ausstoss der Flüssigkeit durch die Nadel führt.

Häufig befindet sich die zu verabreichende Flüssigkeit nicht direkt im Hauptkörper, sondern in einer Ampulle, wobei die Flüssigkeit zwischen einer Durchstechmembrane und einem gleitbeweglichen Kolben gelagert ist.

Je nach Injektionsgerät wird von der Abgabemechanik Unterschiedliches erwartet. Es gibt Geräte, welche lediglich eine einzige Ausschüttung, solche die mehrere gleichgrosse Ausschüttungen und solche, die mehrere frei wählbare Ausschüttungen zulassen.

Unabhängig davon wie komplex die Abgabemechanik aufgebaut ist, ist es bei denjenigen Injektionsgeräten, welche ein Auswechseln der Ampulle zulassen, für den Patienten sehr aufwendig, wenn das Abtriebsglied in die Ausgangsposition zurückgedreht werden muss, um nach dem Auswechseln der Ampulle wieder betriebsbereit zu sein. Geräte, welche eine Rückdrehung des Abtriebsgliedes mittels Bedienknopf verlangen, sind aus der WO 93/16740 bekannt.

Für den Patienten einfacher zu bedienen sind diejenigen Geräte, welche ein Rückschieben der Gewindestange erlauben, wie in den Schriften US-A-4 592 745 und EP-A-0 554 995 offenbart. Nachteilig an diesen bekannten Geräten ist, dass durch Lösung des distalen Bereiches vom proximalen Bereich des Hauptkörpers, die Gewindemutter gespreizt wird und dadurch die Gewindestange frei bewegbar ist, ohne dass dies vom Patienten eine bewusste Handlung verlangt, so dass ein Zusammenschrauben der beiden Bereiche des Hauptkörpers, nach dem Auswechseln der Ampulle, leicht zu einer vorzeitigen, ungewollten Ausschüttung der Flüssigkeit führen kann. Je nach Injektionsgerät kann auch eine Fehldosierung daraus resultieren, was bei gewissen Medikamenten sehr gefährlich für die Gesundheit eines Patienten ist.

Der Erfindung liegt die Aufgabe zugrunde, ein Injektionsgerät mit einem rücksetzbaren Abtriebsglied zu schaffen, bei dem mit größerer Sicherheit als bei den bekannten Geräten eine versehentliche Rücksetzung des Abtriebsglieds verhindert wird.

Die Erfindung löst die Aufgabe mit einem Injektionsgerät, welches die Merkmale des Anspruchs 1 aufweist.

Die durch die Erfindung erreichten Vorteile sind im Wesentlichen darin zu sehen, dass ohne zusätzlich anzubringende Bedienungselemente, ein freies Zurückgleiten des Abtriebsgliedes nur dann möglich ist, wenn der Patient durch Drehen, also mit einer bewussten zusätzlichen Handlung, das Abtriebsglied freigibt.

Ein Ausführungsbeispiel der Erfindung ist in den Figuren dargestellt.

Es zeigen:
Fig. 1 ein erfindungsgemässes Injektionsgerät mit gehaltenem Abtriebsglied;
Fig. 2 ein erfindungsgemässes Injektionsgerät mit einem in Längsrichtung frei beweglichen Abtriebsglied;
Fig. 3 einen Querschnitt durch die Linie A-A in Fig. 1;
Fig. 4 einen Querschnitt durch die Linie B-B in Fig. 1;
Fig. 5 einen Querschnitt durch die Linie C-C in Fig. 1; und
Fig. 6 ein Kupplungsteil für das erfindungsgemässe Injektionsgerät.

In der nachfolgenden Beschreibung werden die Begriffe proximal und distal in der in der Medizin üblichen Weise gebraucht, also
proximal = von dem Patienten abgewandt und distal = dem Patienten zugewandt.

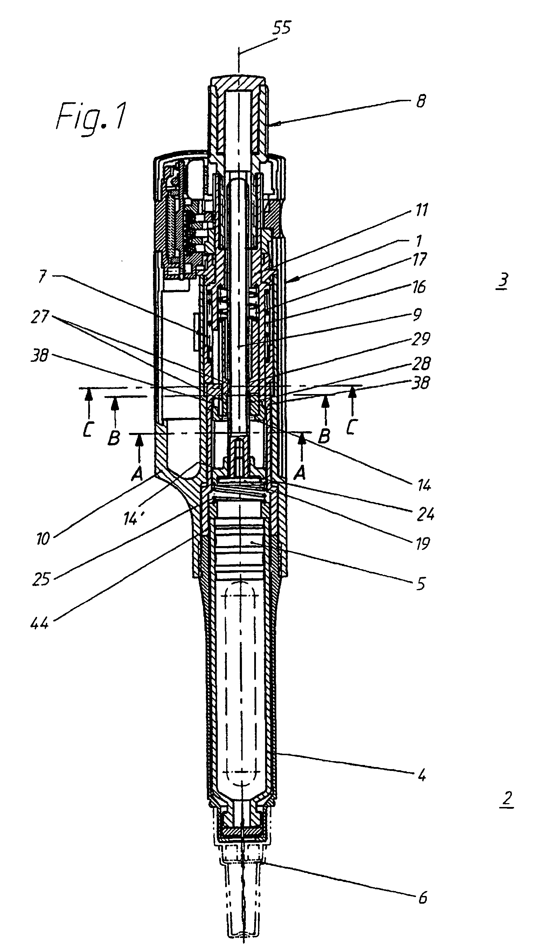

Wie in Fig. 1 dargestellt, besteht das erfindungsgemässe Injektionsgerät aus einem hülsenförmigen Körper 1, der sich in einen hinteren (proximalen) Bereich 3, welcher die Betätigungseinrichtung oder Ausschüttmechanik 7 beinhaltet und einen vorderen (distalen) Bereich 2, der eine austauschbare Karpule 4 mit bewegbarem Kolben 5 beinhaltet, einteilen lässt. Auf dem distalen Ende des Hauptkörpers 1 kann eine Nadel 6 aufgeschraubt werden, deren proximales Ende mit der zu verabreichenden Flüssigkeit in Verbindung steht. Die Betätigungseinrichtung 7 weist einen Bedienknopf 8, ein stabförmiges Abtriebsglied 9 in Form einer Gewindestange mit einem Flansch 19, ein Führungselement 24 und ein hohlzylindrische Gegenelement 11 in Form eines Antriebselementes auf. Das Abtriebsglied 9 wird im folgenden als Gewindestange 9 bezeichnet.

Das röhrenförmige Antriebselement 11 ist mit dem Bedienknopf 8 fest gegen Verdrehung verbunden. Am distalen Ende weist es eine geteilte Gewindemutter 27 auf, welche in das Gewinde der Gewindestange 9 eingreift. Die Gewindestange 9 ist im Inneren des Antriebselementes 11 gelagert.

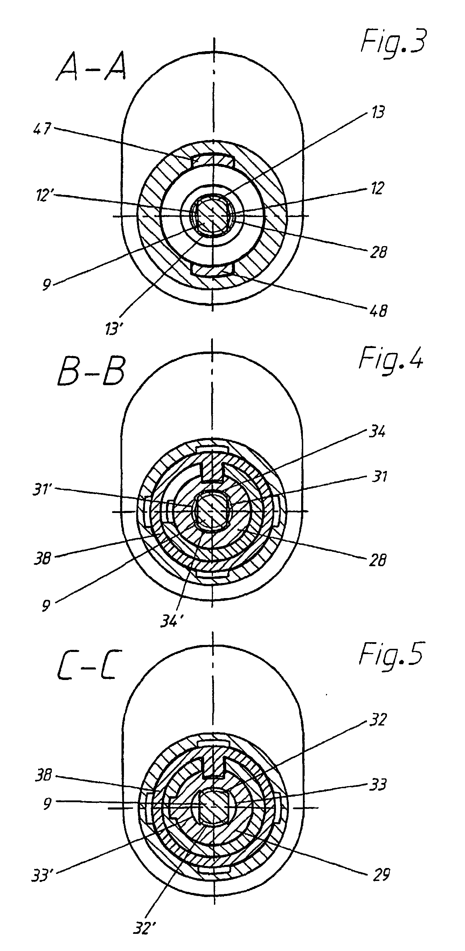

Aus Figur 3 ist ersichtlich, dass die Gewindestange 9 zwei gegenüberliegende, plane Flächen 12,12' besitzt, im übrigen aber einen kreisförmigen Querschnitt aufweist und auf den kreisförmigen Abschnitten 13,13' ein Gewinde trägt.

Das Führungselement 24 ist sowohl gegen Rotationsbewegungen wie auch gegen axiale Verschiebungen fest mit dem Mechanikhalter 10 und somit mit dem hülsenförmigen Körper 1 verbunden und liegt vor dem Antriebselement 11. Die Öffnung im Führungselement 24, durch welche die Gewindestange 9 hindurchgeht, besitzt denselben Querschnitt - um eine bestimmte Toleranz vergrössert, wie der Querschnitt der Gewindestange 9. Da das Führungselement 24 - im Gegensatz zum Antriebselement 11 - kein Gewinde aufweist, kann die Gewindestange 9 durch diese Öffnung des Führungselementes 24 in axialer Richtung hin- und hergeschoben werden. Eine Rotationsbewegung der Gewindestange 9 ist somit nicht möglich, weil dies das Führungselement 24 nicht zulässt.

Am Bedienknopf 8 können sowohl axiale, wie auch rotierende Bewegungen vorgenommen werden. Wird er durch Druck in distaler Richtung bewegt, so verschiebt er gleichzeitig das Antriebselement 11 bis seine vordere Stirnfläche 14 auf die hintere Stirnfläche 14' des Führungselementes 24 auf trifft.

Die Gewindestange 9 ist mit dem Antriebselement 11 durch die Gewindesegmente 31, 31', 32, 32' verbunden, so dass axiale Bewegungen des Bedienknopfes 8 übertragen werden können.

Eine detaillierte Beschreibung des Muttergewindes erfolgt weiter unten.

Die axiale Bewegung wird entgegen der Kraft einer Feder 16 ausgeführt, welche die Betätigungseinrichtung 7 wieder in die Ruhelage zurückbringt.
Wird der Bedienknopf 8 zur Einstellung einer Dosis gedreht, so dreht sich das Antriebselement 11 mit ihm. Diese Drehbewegung wird aber nicht auf die Gewindestange 9 übertragen, da diese im Führungselement 24 drehfest gelagert ist. Durch die sich drehende Gewindemutter 27 des Antriebselements 11 wird über die Gewindesegmente 31,31',32,32' an den Gewindeabschnitten 13,13' der Gewindestange 9 diese drehfest nach vorne (oder bei entgegengesetzter Drehung des Bedienknopfes 8 nach hinten) getrieben, und so der Flansch 19 in diejenige Stellung gebracht, welche die nächste abzugebende Dosis erfordert, d.h. der Abstand des Flansches 19 vom Kolben 5 wird entsprechend verringert.

Durch anschliessenden Druck auf den Bedienknopf 8 wird die voreingestellte Dosis ausgeschüttet. Dieser Vorgang ist im Detail in der WO 93/16740 beschrieben.

Ist die Karpule 4 leer und befindet sich demnach die Gewindestange 9 in ihrer vordersten Position, so muss diese wieder in ihre hinterste Position gebracht werden, bevor eine neue volle Karpule 4 angekoppelt werden kann. Das erfindungsgemässe Injektionsgerät 1 erlaubt nun eine Rückschiebung der Gewindestange 9. Aus den Fig. 1-5 ist ersichtlich, dass das Gewinde des Antriebselements 11 horizontal bezüglich der Längsachse 55 in zwei hülsenförmige Gewindemutterhälften 28,29 geteilt ist. Eine Rückschiebung der Gewindestange 9 wird dadurch ermöglicht, dass die mit den Gewindemutterhälften 28, 29 in Eingriff stehenden Gewindeabschnitte 13,13' der Gewindestange 9 von den Gewindemutterhälften 28,29 gelöst werden. Jede dieser Gewindemutterhälften 28,29 weist zwei Gewindesegmente 31,31',32,32' auf, welche von den glatten Innenflächen 33, 33', 34, 34' vorstehen. Immer zwei Gewindesegmente (90°) 31, 31', 32, 32' liegen sich gegenüber. Jedem 90° Gewindesegment 31,31',32,32' folgt ein 90° glatter Abschnitt 33,33',34,34'. Die beiden Gewindemutterhälften 28,29 sind im Betriebszustand gegeneinander um 90° verschoben, so dass die Gewindestange 9 im Gesamten von 360° Gewinde umgeben wird. Das Aussengewinde der Gewindestange 9 befindet sich demnach zeitweise nur mit zwei Gewindesegmenten der einen oder anderen Gewindemutterhälfte 28, 29 im Eingriff. Wie weiter unten gezeigt wird, ist dieser Eingriff von lediglich zwei Gewindesegmenten in das Aussengewinde der Gewindestange 9 wesentlich für die Möglichkeit der Rückschiebung der Gewindestange 9.

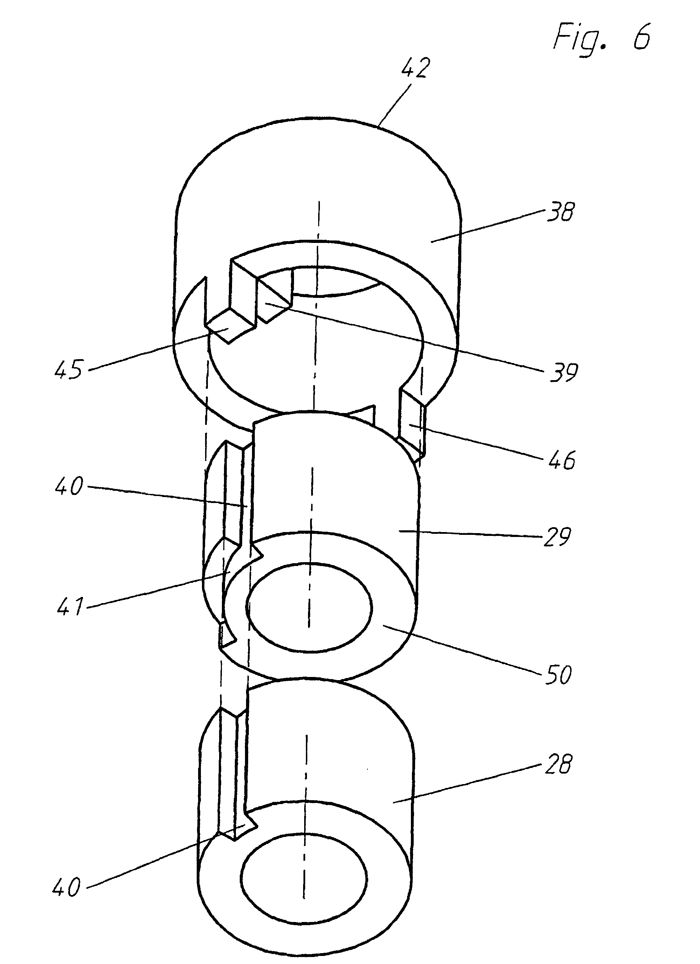

Die beiden hülsenförmigen Gewindemutterhälften 28,29 werden von einem hülsenförmigen Kupplungsteil 38 umgeben. Dabei entspricht der Innendurchmesser des Kupplungsteils 38 dem Aussendurchmesser der Gewindemutterhälften 28,29, so dass das Kupplungsteil 38 über die Gewindemutterhälften 28, 29 gleiten kann. Aus Fig. 6 ist ersichtlich, dass das Kupplungsteil 38 einen Innennocken 39 besitzt, welcher in einen Einschnitt 40 der Aussenflächen der Gewindemutterhälften 28,29 eingreift.

Die proximale Gewindemutterhälfte 29 ist mit dem Antriebselement 11 drehfest verbunden, so dass jede Rotationsbewegung des Antriebselements 11 unmittelbar auf die proximale Gewindemutterhälfte 29 übertragen wird. Diese Bewegung wird mittels dem Kupplungsteil 38 auf die distale Gewindemutterhälfte 28 übertragen. Eine axiale Bewegung des Antriebselements 11 wird über die Feder 17 auf die beiden Gewindemutterhälften 28,29 übertragen.

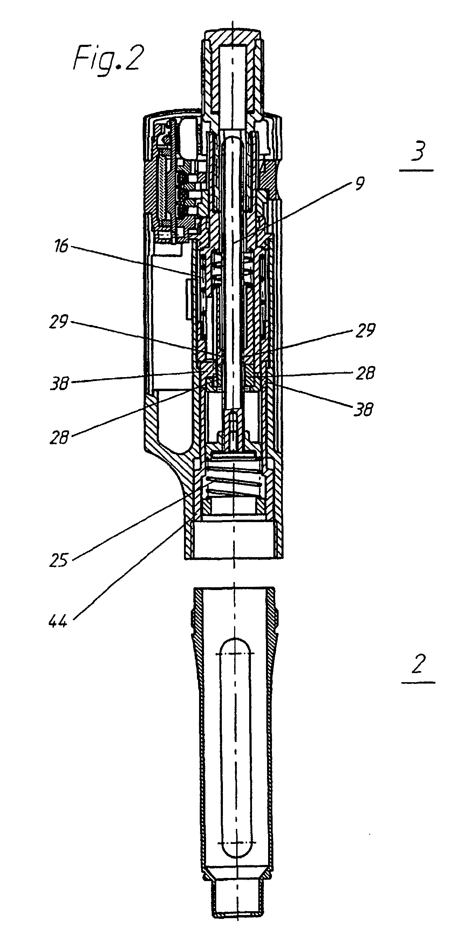

Das Kupplungsteil 38 ist zwischen einer proximalen und einer distalen Position axial verschiebbar. Die proximale Position entspricht der aus Fig. 1 ersichtlichen Betriebsposition, die distale Position dagegen der aus Fig. 2 ersichtlichen Position. Wie unten ausgeführt wird, kann die Gewindestange auch in der in Fig. 2 dargestellten Position noch von der distalen Gewindemutter gehalten sein.

Befindet sich das Kupplungsteil 38 in seiner proximalen Position, sind die beiden Gewindemutterhälften 28,29 um 90° verdreht gekoppelt und damit die Gewindestange 9 von 360° Gewinde umgeben. Befindet sich dagegen das Kupplungsteil 38 in seiner distalen Position, sind die beiden Gewindemutterhälften 28,29 um 90° verdrehbar, d.h. aus dem Eingriff mit der Gewindestange 9 lösbar.

Damit eine Verdrehung der beiden Gewindemutterhälften 28,29 in der distalen Position des Kupplungsteils 38 möglich ist, weist die proximale Gewindemutterhälfte 29 an ihrer distalen Stirnfläche 50 einen gegenüber dem Einschnitt 40 senkrecht verlaufenden Einschnitt 41 auf. Befindet sich das Kupplungsteil 38 in distaler Position, liegt die hintere Stirnfläche 42 und somit der Innennocken 39 im Einschnitt 41 der proximalen Gewindemutterhälfte 29 und ermöglicht dadurch eine Verdrehung der proximalen Gewindemutterhälfte 29 um 90° gegenüber der distalen Gewindemutterhälfte 28.

Die Veränderung der Position des Kupplungsteils 38 wird durch einen Schieber 44 bewirkt.

Befinden sich der Schieber 44 und das Kupplungsteil 38 in proximaler Position (Fig. 1), berühren zwei an der distalen Stirnseite 43 des Kupplungsteils 38 angebrachte Nocken 45,46 den Mechanikhalter 10. Im Mechanikhalter 10 befinden sich zwei Öffnungen 47 und 48, welche durch zwei Verlängerungen des Schiebers 44 aufgefüllt sind und dadurch verhindern, dass die beiden Nocken 45,46 in distale Position gleiten können. Die beiden Öffnungen 47,48 sind so angeordnet, dass das als Kupplungshülse gebildete Kupplungsteil 38 nur dann in die distale Position gleiten kann, wenn die Gewindemuttersegmente 31,31' der distalen Gewindemutterhälfte 28 nicht im Eingriff mit der Gewindestange 9 sind.

Sind die beiden Hauptbereiche 2,3 des Hauptkörpers 1 getrennt (Fig. 2), wird der Schieber 44 durch den Druck der Feder 16 über das Kupplungsteil 38 in seinen vorderen Anschlag geschoben, wenn sich die beiden Nocken 45,46 des Kupplungsteils 38 über den Öffnungen 47,48 des Mechanikhalters 10 befinden. Das Kupplungsteil 38 kann nur in seine distale Position gleiten und gleichzeitig den Schieber 44 in die distale Richtung schieben, wenn sich die beiden Nocken 45,46 des Kupplungsteils 38 über den Öffnungen 47,48 des Mechanikhalters 10 befinden. Befinden sich der Schieber 44 und das Kupplungsteil 38 in distaler Position, führt eine Verdrehung des Bedienknopfes 8 um 90° in Gegenrichtung der normalen Dosierung zu einer Verdrehung der proximalen Gewindemutterhälfte 29 um 90°, d.h. dazu, dass beide Gewindemutterhälften 28,29 hintereinander in derselben Position gelagert sind. In dieser Position ist keines der Gewindemuttersegmente 31, 31', 32 , 32' im Eingriff mit dem Gewinde 13, 13' der Gewindestange 9, mithin die Gewindestange 9 frei verschiebbar.

Werden die beiden Hauptbereiche 2,3 ineinandergefügt und liegen die beiden Gewindemutterhälften 28,29 in um 90° verdrehter Position - demnach fest im Eingriff mit der Gewindestange 9 - kann der Schieber 44 in proximaler Richtung verschoben werden und gleichzeitig das Kupplungsteil 38 in proximaler Richtung schieben, weil der Innennocken 39 des Kupplungsteils 38 im Einschnitt 40 der beiden Gewindemutterhälften 28,29 gleiten kann.

Befinden sich die beiden Gewindemutterhälften 28, 29 dagegen nicht in um 90° verdrehter Position und wird trotzdem versucht, die beiden Hauptbereiche 2,3 ineinanderzufügen, kann der Schieber 44 nicht in proximaler Richtung geschoben werden, weil der Innennocken 39 des Kupplungsteils 38 im Einschnitt 41 der proximalen Gewindemutterhälfte 29 liegt. Es ist deshalb nicht möglich, in einer solchen Position der beiden Gewindemutterhälften 28,29 die beiden Hauptbereiche 2,3 vollständig ineinanderzufügen. Um den Patienten darauf aufmerksam zu machen, dass das Injektionsgerät nicht betriebsbereit ist, kann derjenige Teil der beiden Hauptbereiche, welcher normalerweise nicht sichtbar ist, aber durch das unvollständige Ineinanderfügen sichtbar bleibt, grell verfärbt werden.

Durch Drehen am Bedienknopf 8 in Betätigungsrichtung wird der Innennocken 39 des Kupplungsteils 38 aus dem waagrechten Einschnitt 41 der proximalen Gewindemutterhälfte 29 in den senkrechten Einschnitt 40 derselben gedreht. In dieser Position kann das Kupplungsteil 38 vom Schieber 44 in die Betriebsposition geschoben werden.

## Patentansprüche

1. Injektionsgerät zum Injizieren von Flüssigkeiten aus einem mit einem Kolben (5) ausgerüsteten Flüssigkeitsbehälter (4)
a) mit einer Betätigungseinrichtung (7), die ein stabförmiges Abtriebsglied (9) aufweist, das auf zwei gegenüberliegenden Seiten plane Flächen (12, 12'), im übrigen aber Kreisflächen (13, 13') mit Gewinden aufweist,
b) mit einem Bedienknopf (8), an dem axiale Bewegungen ausführbar sind,
c) und mit einem zum stabförmigen Abtriebsglied (9) passenden, dazu koaxial angeordneten, hohlzylindrischen Gegenelement (11) mit geteilter hülsenförmiger Gewindemutter (27), die mit dem Abtriebsglied (9) in einem Gewindeeingriffist,
**dadurch gekennzeichnet, dass**
d) die Gewindemutter (27) quer zur Längsachse geteilt ist,
e) jeder Teil (28, 29) der Gewindemutter (27) zwei gegenüberliegende Gewindesegmente (31, 31', 32, 32') aufweist und zwischen den Gewindesegmenten (31, 31', 32, 32') gewindefreie Flächen (33, 33', 34, 34') liegen, so dass bei Verdrehung der hülsenförmigen Gewindemutterteile (28, 29) gegeneinander das Abtriebsglied (9) frei verschiebbar ist,
f) und dass ein axial verschiebbares, hülsenförmiges Kupplungsteil (38) vorgesehen ist, das in einer ersten Axialposition die Gewindemutterteile (28, 29) drehsteif miteinander koppelt und in einer zweiten Axialposition die Verdrehung der Gewindemutterteile (28, 29) gegeneinander zulässt.

2. Injektionsgerät nach Anspruch 1, **dadurch gekennzeichnet, dass** die Verdrehung des Gegenelementes (11) am Bedienknopf (8), welcher mit dem Abtriebsglied (11) drehfest verbunden ist, vorgenommen wird.

3. Injektionsgerät nach Anspruch 1, **dadurch gekennzeichnet, dass** ein zusätzliches Bedienelement eine Verdrehung des Gegenelements (11) ermöglicht, so dass das freie Verschieben des Abtriebsgliedes (9) ermöglicht wird.

4. Injektionsgerät nach einem der vorhergehenden Ansprüche, **dadurch gekennzeichnet, dass** das Gegenelement (11) die Gewindemutter (27) am distalen Ende aufweist.

5. Injektionsgerät nach einem der vorhergehenden Ansprüche, **dadurch gekennzeichnet, dass** beide Teile (28, 29) der hülsenförmigen Gewindemutter (27) für die Kopplung mit dem Kupplungsteil (38) an der Außenseite einen parallel zur Längsachse verlaufenden Einschnitt (40) und der proximale Gewindemutterteil (28) an seiner distalen Kante (50) zusätzlich einen, um ein Viertel des Hülsenumfangs in Umfangsrichtung verlaufenden Einschnitt (41) aufweisen.

6. Injektionsgerät nach einem der vorhergehenden Ansprüche, **dadurch gekennzeichnet, dass** das Kupplungsteil (38) mit einem Innennocken (39) versehen ist, dessen Hinterkante (42), in der zweiten, distalen Position des Kupplungsteils (38) derart mit dem waagrechten Einschnitt (41) des proximalen Gewindemutterteils (29) verbunden ist, dass die Verdrehung der beiden Teile (28, 29) der Gewindemutter (27) gegeneinander möglich ist.

7. Injektionsgerät nach einem der vorhergehenden Ansprüche, **dadurch gekennzeichnet, dass** das Kupplungsteil (38) nur in einer bestimmten Drehwinkelposition axial verschiebbar ist.

8. Injektionsgerät nach einem der vorhergehenden Ansprüche, **dadurch gekennzeichnet, dass** das Injektionsgerät ein Gehäuse (1) mit einem distalen Gehäusebereich (2), der die zu injizierende Flüssigkeit und einen bewegbaren Kolben (5) zum Verdrängen der Flüssigkeit beinhaltet, und einem proximalen Gehäusebereich (3) aufweist, der die Betätigungseinrichtung (7), den Bedienknopf (8), das Abtriebsglied (9), das Gegenelement (11) und das Kupplungsteil (38) beinhaltet, dass die Gehäusebereiche (2, 3) voneinander lösbar sind und dass das Kupplungsteil (38) die zweite Axialposition nur einnehmen kann, wenn die Gehäusebereiche (2, 3) voneinander gelöst sind.

## Claims

1. An injection device for injecting liquids from a liquid container (4) fitted with a piston (5), comprising:
a) an activating means (7) which comprises a rod-shaped driven member (9) which has plane surfaces (12, 12') on two opposing sides but otherwise has circular surfaces (13, 13') having threads;
b) an operating button (8) on which axial movements can be performed;
c) and a hollow-cylindrical counter element (11) which fits said rod-shaped driven member (9) and is arranged co-axially with respect to it, and comprises a split, sleeve-shaped threaded nut (27) which is in threaded engagement with the driven member (9);
**wherein:**
d) said threaded nut (27) is split perpendicular to the longitudinal axis;
e) each part (28, 29) of the threaded nut (27) comprises two opposing threaded segments (31, 31', 32, 32') and there are unthreaded surfaces (33, 33', 34, 34') between the threaded segments (31, 31', 32, 32'), such that when the sleeve-shaped threaded nut parts (28, 29) are rotated with respect to each other, the driven member (9) can be freely shifted;
f) and an axially shifting, sleeve-shaped coupling part (38) is provided which, in a first axial position, couples the threaded nut parts (28, 29) to each other, secured against rotation, and in a second axial position allows the threaded nut parts (28, 29) to rotate with respect to each other.

2. The injection device as set forth in claim 1, wherein said counter element (11) is rotated on said operating button (8) which is connected, secured against rotation, to the driven member (9).

3. The injection device as set forth in claim 1, wherein an additional operating element enables the counter element (11) to be rotated such that the driven member (9) can be freely shifted.

4. The injection device as set forth in any one of the preceding claims, wherein the counter element (11) comprises the threaded nut (27) at the distal end.

5. The injection device as set forth in any one of the preceding claims, wherein for coupling to the coupling part (38), both parts (28, 29) of the sleeve-shaped threaded nut (27) comprise a notch (40), running parallel to the longitudinal axis, on their outer side, and the proximal threaded nut part (28) additionally comprises a notch (41), running a quarter of the circumference of the sleeve in the circumferential direction, on its distal edge (50).

6. The injection device as set forth in any one of the preceding claims, wherein the coupling part (38) is provided with an inner cam (39) whose rear edge (42) is connected to the horizontal notch (41) of the proximal threaded nut part (29) in the second, distal position of the coupling part (38), such that the two parts (28, 29) of the threaded nut (27) can be rotated with respect to each other.

7. The injection device as set forth in any one of the preceding claims, wherein the coupling part (38) can only be axially shifted in a particular rotational angular position.

8. The injection device as set forth in any one of the preceding claims, wherein: the injection device comprises a casing (1) including a distal casing portion (2) which contains the liquid to be injected and a movable piston (5) for displacing the liquid, and a proximal casing portion (3) which contains said activating means (7), the operating button (8), the driven member (9), the counter element (11) and the coupling part (38); the casing portions (2, 3) can be released from each other; and the coupling part (38) can only assume the second axial position when the casing portions (2, 3) have been released from each other.

## Revendications

1. Appareil d'injection pour injecter des liquides à partir d'un récipient (4) équipé d'un piston (5), comprenant
a) un dispositif d'actionnement (7) muni d'un organe de sortie (9) en forme de tige présentant, sur deux côtés opposés, des surfaces planes (12, 12') et, sur sa partie restante, des surfaces circulaires (13, 13') dotées de filetages,
b) un bouton de manoeuvre (8) permettant l'exécution de mouvements axiaux,
c) et un élément complémentaire (11) cylindrique creux, s'adaptant à l'organe de sortie (9) en forme de tige, agencé coaxialement à ce dernier et pourvu d'un écrou scindé (27) en forme de douille, en prise par filetage avec ledit organe de sortie (9),
**caractérisé par le fait que**
d) l'écrou (27) est scindé transversalement par rapport à l'axe longitudinal,
e) chaque partie (28, 29) dudit écrou (27) comporte deux segments filetés opposés (31, 31', 32, 32'), des surfaces (33, 33', 34, 34') exemptes de filetage étant interposées entre lesdits segments filetés (31, 31', 32, 32'), de façon telle que l'organe de sortie (9) puisse coulisser librement lorsque les parties (28, 29) de l'écrou, configurées en des douilles, sont animées d'une rotation l'une en direction de l'autre,
f) et qu'il est prévu une pièce d'accouplement (38) en forme de douille pouvant coulisser axialement qui, dans une première position axiale, provoque l'accouplement des parties (28, 29) de l'écrou sans faculté de rotation et, dans une seconde position axiale, autorise la rotation réciproque desdites parties (28, 29) de l'écrou.

2. Appareil d'injection selon la revendication 1, **caractérisé par le fait que** la mise en rotation de l'élément complémentaire (11) s'opère sur le bouton de manoeuvre (8) relié à l'organe de sortie (11) avec verrouillage rotatif.

3. Appareil d'injection selon la revendication 1, **caractérisé par le fait qu'**un élément supplémentaire d'actionnement autorise une rotation de l'élément complémentaire (11), ce qui rend possible le libre coulissement de l'organe de sortie (9).

4. Appareil d'injection selon l'une des revendications précédentes, **caractérisé par le fait que** l'élément complémentaire (11) présente l'écrou (27) à l'extrémité distale.

5. Appareil d'injection selon l'une des revendications précédentes, **caractérisé par le fait que** les deux parties (28, 29) de l'écrou (27) en forme de douille présentent à la face extérieure, en vue de l'accouplement avec la pièce d'accouplement (38), une saignée (40) s'étendant parallèlement à l'axe longitudinal ; et la partie proximale (28) dudit écrou est additionnellement pourvue, sur son bord distal (50), d'une saignée (41) s'étendant périphériquement sur un quart du pourtour de la douille.

6. Appareil d'injection selon l'une des revendications précédentes, **caractérisé par le fait que** la pièce d'accouplement (38) est dotée d'un mentonnet intérieur (39) dont le bord postérieur (42) est relié à la saignée horizontale (41) de la partie proximale (29) de l'écrou, dans la seconde position distale de la pièce d'accouplement (38), de manière que les deux parties (28, 29) de l'écrou (27) puissent accomplir une rotation l'une en direction de l'autre.

7. Appareil d'injection selon l'une des revendications précédentes, **caractérisé par le fait que** la pièce d'accouplement (38) ne peut être animée d'un coulissement axial que dans une position angulaire déterminée.

8. Appareil d'injection selon l'une des revendications précédentes, **caractérisé par le fait que** ledit appareil d'injection comprend un boîtier (1) composé d'une région distale (2) qui renferme le liquide à injecter, et un piston mobile (5) conçu pour refouler ledit liquide, et d'une région proximale (3) renfermant le dispositif d'actionnement (7), le bouton de manoeuvre (8), l'organe de sortie (9), l'élément complémentaire (11) et la pièce d'accouplement (38) ; **par le fait que** les régions (2, 3) dudit boîtier peuvent être dissociées l'une de l'autre ; et **par le fait que** ladite pièce d'accouplement (38) ne peut prendre la seconde position axiale que lorsque lesdites régions (2, 3) du boîtier sont dissociées l'une de l'autre.
